# FASCICULE DE BREVET EUROPEEN

(11) **EP 2 296 992 B1**
(45) Date de publication et mention de la délivrance du brevet: **20.06.2012**
(21) Numéro de dépôt: 09734029.3
(22) Date de dépôt: 27.03.2009
(51) Int. Cl.: B65D 47/20, A61F 9/00

(54) **DISPOSITIF DE DISTRIBUTION DE LIQUIDE CONTENU DANS UN RESERVOIR**
VORRICHTUNG ZUR AUSGABE VON IN EINEM TANK ENTHALTENER FLÜSSIGKEIT
DEVICE FOR DISPENSING A LIQUID CONTAINED IN A TANK

(30) Priorité: 27.03.2008 FR 0851994
(43) Date de publication de la demande: 23.03.2011
(73) Titulaire: Rexam Healthcare La Verpillière, 38290 La Verpillière (FR)
(72) Inventeur: PAINCHAUD, Gaétan, 69340 Francheville (FR); LANZI , Sylvain, Chirens 38850 (FR); JULIA, Xavier, 38090 Villefontaine (FR)
(74) Mandataire: Thiollier, Clémence-Olivia Laure Marie
(86) Numéro de dépôt international: PCT/FR2009/000357
(87) Numéro de publication internationale: WO 2009/130412

(56) Documents cités:
- WO-A-2006/119315
- JP-A- 2001 240 088
- US-A1- 2004 134 940

## Description

La présente invention concerne le domaine de la distribution de liquide contenu dans un réservoir. Plus particulièrement mais non exclusivement, l'invention concerne la distribution de doses prédéterminées de liquide, par exemple pour une application oculaire, nasale ou buccale, notamment la distribution de gouttes de collyre.

On connaît déjà des flacons contenant un liquide tel que du collyre et munis d'un système de distribution du liquide sous forme de gouttes. Souvent, il est nécessaire de conserver le liquide sans risque de contamination par des agents extérieurs tels que des bactéries. Un moyen simple d'assurer une conservation stérile du liquide consiste à utiliser des flacons uni-dose, jetables après leur première utilisation. Néanmoins, l'utilisation de flacons jetables est gênante du point de vue écologique.

De façon alternative au flacon uni-dose, on peut utiliser un flacon destiné à plusieurs utilisations, que l'on peut refermer une fois une dose distribuée, afin de distribuer ultérieurement d'autres doses de liquide. La difficulté de l'utilisation d'un flacon multi-dose réside dans la conservation du liquide stocké. En effet, à chaque distribution d'une dose, des agents extérieurs risquent de s'introduire dans le réservoir, et donc de contaminer le liquide stocké. Afin d'assurer la non contamination du liquide, on ajoute généralement des agents conservateurs chimiques dans le liquide. Néanmoins, on constate que l'utilisation de tels agents conservateurs peut faire apparaître des allergies chez certains utilisateurs, ou encore des effets secondaires indésirables.

Le document US 2004/0134940 A1 divulgue un dispositif de distribution qui possède les caractéristiques définies au préambule de la revendication 1.

La présente invention vise à proposer un dispositif de distribution du liquide utilisable plusieurs fois, assurant de façon satisfaisante la non contamination du liquide conservé, sans pour autant nécessiter d'ajouter des agents conservateurs dans le liquide.

A cet effet, l'invention a pour objet un dispositif de distribution de liquide contenu dans un réservoir, ayant les caractéristiques définies à la revendication 1.

Ainsi, on propose que la position anti-retour de l'élastomère mette en oeuvre une déformation de l'élastomère, si bien que l'élastomère cherche à retrouver sa forme initiale (non déformée), ce qui permet d'exercer une contrainte sur l'élastomère, et ainsi de le faire plaquer contre un autre élément, afin d'assurer l'étanchéité du dispositif en position anti-retour. Il en résulte que le liquide contenu dans le réservoir, une fois sorti d'une zone étanche du dispositif, ne peut pas se réintroduire à nouveau dans cette zone étanche, laquelle est fermée par l'élément élastomère plaqué de façon étanche contre un autre élément du dispositif. Grâce à cet agencement, le liquide contenu dans le réservoir ne peut passer que dans un sens, il peut uniquement sortir du réservoir. En d'autres termes, l'élément élastomère assure une étanchéité dynamique du dispositif.

On notera que la déformation de l'élément l'élastomère est de préférence une déformation par flexion, c'est-à-dire que les moyens d'appui exercent une force localement sur l'élément élastomère, au droit d'une zone évidée, de façon à le faire fléchir. Du fait de ce fléchissement, l'élément élastomère cherche à retrouver sa forme initiale et reste donc plaqué localement contre les moyens d'appui, ce qui assure l'étanchéité désirée. On notera que la déformation est générée par un appui sur l'élément élastomère au droit d'une zone évidée, c'est-à-dire qu'un espace est prévu derrière la partie de l'élastomère sur laquelle on appuie pour permettre sa déformation, plus précisément pour faire fléchir l'élastomère. La déformation se distingue donc d'une seule compression de l'élément élastomère contre une surface dure. Ainsi, outre une possible compression générale de l'élément l'élastomère, on crée grâce aux moyens d'appui une déformation locale, modifiant la forme générale de l'élément élastomère.

Parmi les avantages du dispositif, on comprendra qu'il est utilisable sur des réservoirs multi-dose et assure au moins en partie la non contamination du liquide stocké dans le réservoir. De façon avantageuse, le liquide est dépourvu d'agents conservateurs, l'étanchéité procurée par l'élément élastomère étant suffisante pour empêcher sa contamination. Bien sûr, on peut aussi prévoir des agents conservateurs dans le liquide.

L'étanchéité proposée par le dispositif est intéressante car elle ne génère pas de contrainte pour l'utilisateur. Elle se distingue par exemple de l'utilisation d'une membrane filtrante, munie d'un filtre, destinée à empêcher que des saletés s'introduisent dans le réservoir. Cette membrane filtrante, bien qu'intéressante pour assurer la non contamination du réservoir, constitue un frein à la délivrance de doses de liquide, ce qui n'est pas confortable pour l'utilisateur qui doit appliquer une force plus grande sur le dispositif pour distribuer du liquide. De préférence, le dispositif présenté ci-dessus ne présente pas un tel freinage à la délivrance de doses, la position de libération pouvant laisser passer le liquide sans le freiner particulièrement.

On notera que l'élément élastomère peut être réalisé en caoutchouc, en silicone, en TPE (thermoplastique élastomère) ou dans tout autre matériau susceptible d'assurer une étanchéité.

Dans la suite, la direction "vers le bas" désigne la direction du réservoir de liquide du dispositif, et la direction "vers le haut" désigne la direction opposée.

L'élément élastomére est chaussé autour du noyau intérieur du dispositif.

Ainsi, ce noyau peut porter des moyens d'appui tels qu'une saillie deformant localement l'élément élastomére, de façon a le plaquer de façon étanche contre les moyens d'appui.

Le dispositif de distribution peut en outre comporter l'une ou plusieurs des caractéristiques définis dans les revendications dependantes 2-9. Entre autres:
- L'élément élastomère, dans sa position de libération de liquide, subit une déformation correspondant à une amplification de la déformation assurée dans sa position anti-retour. Ainsi, l'élément élastomère prend sa position de libération uniquement a la suite d'une pression suffisante pour le déformer davantage, telle que celle exercée par l'utilisateur sur le réservoir afin de faire sortir du fluide, si bien qu'il n'est pas possible que l'élément élastomére prenne cette position de libération de liquide sans cette pression imposée par l'utilisateur. Une telle configuration de l'élastomère assure une bonne étanchéité dynamique, laissant passer le liquide dans un seul sens.
- L'élément élastomère comprend un cône incurvé vers le haut, chaussé sur le noyau interne du dispositif.
- Le dispositif comporte un canal de guidage du liquide, guidant le liquide libéré par l'élément élastomère hors du dispositif.
- L'élément élastomère comporte des moyens de dosage du liquide à distribuer. Ces moyens de dosage délimitent par exemple une cavité dont la géométrie permet de former des gouttes de liquide calibrées pour qu'elles aient toujours le même volume. Il est particulièrement avantageux d'intégrer ces moyens de dosage directement dans l'élément élastomère, du fait que le liquide libéré par l'élément élastomère en position de libération, sortant ainsi de la zone étanche, s'écoule uniquement à l'intérieur de l'élément élastomère. Il en résulte que l'on diminue les risques que le liquide s'infiltre dans d'autres parties du dispositif après sa libération. En effet, dans le cas où les moyens de dosage sont prévus sur une autre pièce que sur l'élément élastomère, par exemple sur une enveloppe recouvrant l'élément élastomère, le liquide peut s'infiltrer dans d'autres pièces du dispositif lors de sa circulation entre l'élément élastomère et les moyens de dosage. Par ailleurs, l'intégration des moyens de dosage dans l'élément élastomère permet de diminuer un volume mort susceptible de contenir du liquide contaminé, correspondant au volume situé entré la zone étanche et les moyens de dosage, délimité ici par l'élément élastomère. En effet, l'élément élastomère, souple, peut avoir une forme qui colle aux pièces et diminue le plus possible ce volume mort, ce qui est plus facile à réaliser que lorsque le volume mort est délimité par des pièces rigides.

L'invention sera mieux comprise à la lecture de la description qui va suivre, donnée uniquement à titre d'exemple et faite en se référant aux dessins dans lesquels :

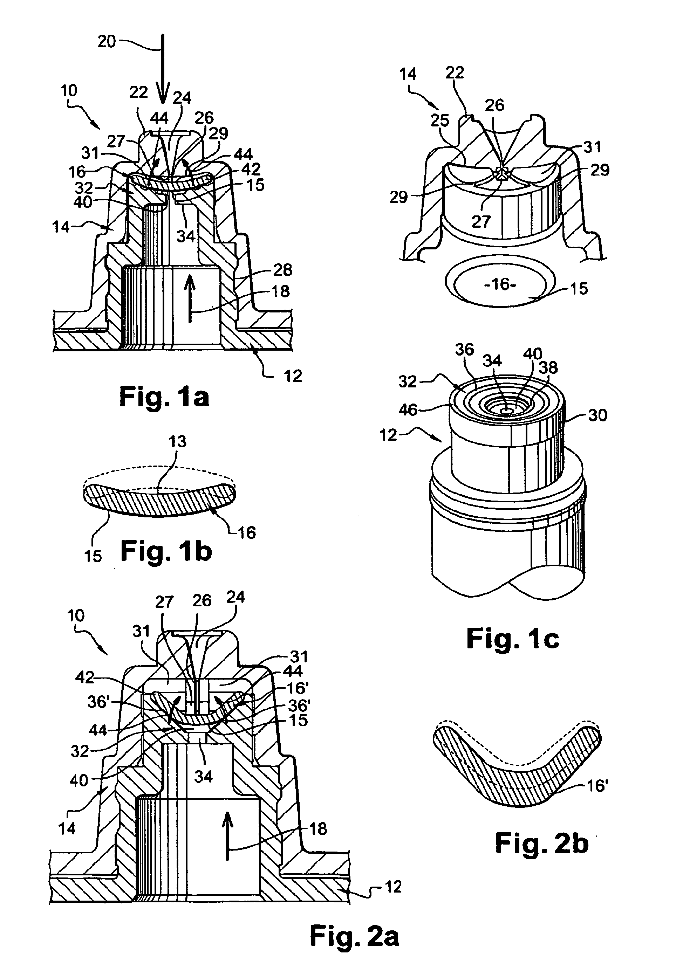

- la figure 1a est une vue en coupe schématique d'un dispositif de distribution selon un premier mode de réalisation qui n'est pas couvert par les revendications, comportant un élément élastomère représenté en position anti-retour;
- la figure 1 b est une vue en coupe schématique de l'élément élastomère de la figure 1a, la configuration non déformée de l'élément étant illustrée en pointillé ;
- la figure 1c est une vue en perspective éclatée d'un noyau intérieur et de l'élément élastomère non déformé du dispositif de la figure 1a, et une vue en coupe et en perspective d'une enveloppe du dispositif de la figure 1a ;
- la figure 2a est une vue similaire à celle de la figure 1a, d'un dispositif selon une variante de réalisation qui n'est pas couverte par les revendications;
- la figure 2b est une vue similaire à la figure 1b, de l'élément élastomère du dispositif de la figure 2a ;
- la figure 3a est une vue similaire à la figure 1a, d'un dispositif selon un deuxième mode de réalisation conforme à l'invention ;
- la figure 3b est une vue similaire à la figure 1 b, de l'élément élastomère du dispositif de la figure 3a ; et
- la figure 3c est une vue en perspective éclatée d'un noyau du dispositif de la figure 3a, une vue en perspective et en écorché de l'élément élastomère du dispositif de la figure 3a, et une vue en coupe et en perspective d'une enveloppe du dispositif de la figure 3a.

Comme on peut le voir sur la figure 1a, un dispositif de distribution 10 selon un premier mode de réalisation comprend un noyau intérieur 12, destiné à être rapporté sur un réservoir (non représenté) contenant un liquide distribué par le dispositif 10. Ce liquide est par exemple du collyre. Le dispositif 10 comporte en outre une enveloppe extérieure 14, pouvant éventuellement être recouverte par un bouchon de protection (non représenté). Entre le noyau 12 et l'enveloppe 14 est intercalé un élément élastomère 16, par exemple une membrane en silicone, en caoutchouc ou encore en TPE (thermoplastique élastomère), ayant une première face 13, dite supérieure, et une seconde face opposée 15, dite inférieure. Cet élément élastomère 16 assure une étanchéité dynamique dans le dispositif 10, c'est-à-dire qu'il autorise le passage de liquide depuis le réservoir vers l'extérieur du dispositif, dans le sens indiqué par la flèche 18, mais qu'il interdit le passage de liquide depuis l'extérieur du dispositif vers l'intérieur du dispositif, c'est-à-dire dans le sens indiqué par la flèche 20.

L'enveloppe extérieure 14 comporte une extrémité 22, dite extrémité de libération, dans laquelle est formé un orifice de libération de liquide hors du dispositif 10. Juste en amont de cet orifice 22, l'enveloppe 14 comprend des moyens 24 de dosage de liquide à distribuer. Ces moyens de dosage 24 comprennent une cavité, dont le volume permet de doser la quantité de liquide à distribuer au cours d'une utilisation, c'est-à-dire au cours d'une certaine pression du réservoir par l'utilisateur. Par ailleurs, la cavité des moyens de dosage 24 a une forme particulière, cette géométrie permettant de donner une forme de goutte à la dose de liquide distribué. En amont des moyens de dosage 24, l'enveloppe 14 comporte un orifice de distribution 26, débouchant sur une surface intérieure 25 de l'extrémité 22 de l'enveloppe. Cette surface 25 comporte, au voisinage de l'orifice d'écoulement 26, des saillies 27, servant de premiers moyens d'appui sur la première face 13 de l'élément élastomère 16. Plus précisément, les premiers moyens d'appui 27 comprennent quatre plots de maintien, séparés les uns les autres par quatre canaux 29, réalisés dans la paroi interne 25, servant de guidage de liquide libéré par l'élément élastomère, comme cela est décrit dans la suite. Les canaux 29 sont espacés à 90° les uns des autres, si bien qu'ils sont, dans cet exemple, alignés deux à deux. Ainsi, sur la figure 1a, la coupe de la figure passe par le centre de deux canaux alignés 29. Comme on peut le voir sur la figure 1a, les saillies 27 réalisées dans la paroi intérieure 25 de l'enveloppe délimitent une première zone évidée, ou évidement 31, constituant un espace entre une partie de l'élément élastomère 16 et l'enveloppe 14. L'enveloppe 14 comporte par ailleurs des moyens 28 d'assemblage sur le noyau intérieur 12. Plus précisément, les moyens 28 comprennent une forme pouvant assurer un encliquetage serré, par exemple une saillie 28 destinée à coopérer avec des moyens complémentaires 30 réalisés dans le noyau 12, par exemple une collerette 30 représentée sur la figure 1c. Les moyens d'assemblage 28, 30 peuvent prendre d'autres formes, l'enveloppe 14 et le noyau 12 pouvant également être solidarisés par serrage ou encore par soudure aux ultrasons.

Le noyau intérieur 12 a une forme, dans cet exemple, de cylindre creux, comportant à son extrémité 32 un orifice 34 de libération du liquide hors du noyau. L'extrémité 32 du noyau intérieur 12 est munie d'une zone d'appui de l'élément élastomère 16. Plus précisément, cette zone d'appui comprend deux saillies 36, 38, circulaires, servant de seconds moyens d'appui, destinés à appuyer sur la seconde face 15 de l'élément élastomère 16. Les moyens d'appui 36, 38 sont disposés en quinconce par rapport aux moyens d'appui 27, c'est-à-dire qu'ils ne sont pas disposés au droit des moyens 27. La surface 32 comporte par ailleurs, en son centre, une zone évidée, ou évidement 40, dans lequel l'orifice 34 débouche.

Comme on peut le voir sur la figure 1c, l'élément élastomère 16 a une forme de disque incurvé. Lorsque l'élément élastomère 16 est en position initiale, c'est-à-dire lorsqu'il n'est pas déformé, il a la forme représentée en pointillé sur la figure 1b ou 1c, incurvée vers le haut. Outre cette position non déformée, l'élément élastomère 16 peut prendre deux autres positions, à savoir une position anti-retour, représentée sur la figure 1a, et en trait plein sur la figure 1b, dans laquelle sa forme générale est la forme d'un disque incurvé vers le bas, et une position de libération de liquide, dans laquelle la forme de l'élément 16 est davantage incurvée vers le bas, la périphérie 42 de l'élément 16 étant alors déplacée vers le haut, conformément aux flèches 44, afin de laisser passer du liquide entre les saillies 36, 38 et cette partie périphérique 42.

Le noyau intérieur 12 et l'enveloppe extérieure 14 sont réalisés de préférence en matière plastique, par exemple en polypropylène ou en polyéthylène.

L'assemblage du dispositif 10 va à présent être décrit, en référence à la figure 1c. Les éléments 12, 14 et 16 sont tout d'abord fabriqués, de façon séparée. Puis, l'élément élastomère 16, dans sa position initiale non déformée, est positionné sur la surface 32 du noyau 12, grâce à des moyens de centrage 46 réalisés sur l'extrémité 32. Une fois l'élément élastomère 16 positionné sur le noyau 12, on rapporte l'enveloppe extérieure 14 par-dessus, de façon à faire coopérer les moyens d'assemblage 28, 30. L'assemblage du noyau 12 et de l'enveloppe 14 déforme l'élément élastomère 16, qui prend ainsi sa position anti-retour visible sur la figure 1a. Dans cette position anti-retour, les premiers moyens d'appui 27, agencés au droit de la zone évidée 40 de l'enveloppe 12, assurent la déformation de l'élément élastomère, plus précisément appuient sur le centre de l'élément élastomère 16 pour le faire fléchir vers le bas, à l'intérieur de l'évidement 40. En parallèle, les seconds moyens d'appui 36, 38, agencés au droit des évidements 31, assurent la déformation de l'élément élastomère en appuyant sur sa partie périphérique 42, de façon que, avec les moyens 27, l'élément 16 fléchisse et que sa concavité s'inverse, comme représenté en trait plein sur la figure 1b, afin qu'il soit plaqué par les premiers moyens 27 contre les seconds moyens 36, 38, et assure ainsi l'étanchéité.

Le fonctionnement du dispositif 10 va à présent être décrit. Dans la position de repos du dispositif, l'utilisateur n'exerce pas de pression sur le réservoir, si bien que le liquide contenu dans ce réservoir reste à l'intérieur du réservoir. Dans cette position, visible sur la figure 1a, comme cela a été décrit précédemment, l'élément 16 est en position anti-retour, les moyens d'appui 27, 36, 38 assurant sa déformation en le plaquant contre les saillies 36, 38, de façon à empêcher que du liquide s'infiltre à l'intérieur du réservoir, plus précisément à l'intérieur de l'orifice d'écoulement 40.

Dans cette position anti-retour, les moyens d'appui 36, 38 contre lesquels est plaquée la partie périphérique 42 de l'élément 16 ferment une zone, appelée zone étanche, à l'intérieur de laquelle les impuretés, telles que des bactéries, ne peuvent pas s'infiltrer, ou du moins s'infiltrent très faiblement, sans que cela soit gênant par rapport aux contraintes de conservation du liquide contenu dans le dispositif 10. La zone étanche comprend dans cet exemple le volume intérieur du réservoir, le volume intérieur du noyau 12, l'orifice 34 et l'évidement 40.

Lorsque l'utilisateur exerce une pression sur le réservoir, du liquide, éventuellement précédé d'air, exerce une pression sur l'élément élastomère 16, qui se déforme davantage dans la direction indiquée par les flèches 44, si bien que le liquide peut passer dans l'espace situé entre les saillies 36, 38 et la face inférieure 15 de l'élément élastomère 16. Le liquide ainsi libéré contourne l'élément élastomère, en passant au bord de sa périphérie 42, et s'infiltre entre sa face supérieure 13 et la surface 25 de l'enveloppe 14. Plus précisément, le liquide s'infiltre à l'intérieur des canaux 29, puis entre les plots 27, et s'échappe ensuite par l'orifice de libération 26, afin de remplir les moyens de dosage 24. Une fois les moyens de dosage 24 remplis, une goutte de liquide peut se former et être libérée hors du dispositif 10. Lorsque l'utilisateur arrête d'exercer une pression, le liquide contenu dans le réservoir n'exerce plus de pression sur l'élément élastomère 16, si bien que ce dernier n'est plus contraint à se déformer pour rester dans sa position de libération de liquide, et reprend donc sa position anti-retour, dans laquelle la partie périphérique 42 est en appui contre les saillies 36, 38.

On notera que le dispositif des figures 1a à 1c est particulièrement intéressant du fait que l'élément 16 est pris en sandwich entre le noyau 12 et l'enveloppe 14, et que très peu d'espace est prévu entre l'élastomère 16 et la surface intérieure de l'enveloppe 14. Il en résulte que peu de place est laissée à l'intérieur du dispositif pour que du liquide ayant passé l'orifice 26, étant donc susceptible de contaminer l'intérieur du dispositif, puisse passer à nouveau dans l'ouverture 26, dans le sens inverse, indiqué par la flèche 20.

Sur la variante de réalisation représentée sur les figures 2a, 2b, l'élément élastomère 16' a une forme générale de cône, incurvé vers le bas. Par ailleurs, le noyau intérieur 12 comprend, sur son extrémité 32, une forme en entonnoir prolongeant l'orifice de libération 34, cette forme en entonnoir présentant une surface d'appui 36', servant de second moyen d'appui sur l'élément élastomère 16', plus précisément sur la partie périphérique 42 de cet élément 16'.

Dans cette variante, l'enveloppe extérieure 14 n'est pas forcément munie de canaux de guidage du liquide 29 vers l'orifice 26, la surface supérieure 13 de l'élément élastomère 16' étant suffisamment incurvée pour guider le liquide vers l'extrémité inférieure de l'orifice 26.

On notera que les surfaces d'appui 36' pourraient éventuellement être munies de saillies telles que les nervures 36, 38 de la figure 1 c.

Le procédé d'assemblage du dispositif 10 de la figure 2a est analogue au procédé d'assemblage illustré sur la figure 1 c. Dans cette variante, les parois en entonnoir de l'extrémité 32 font office de moyens de centrage 46, si bien que, avant de rapporter l'enveloppe extérieure 14, l'élément élastomère 16' dans sa position non déformée (représentée en pointillé sur la figure 2b) est facilement positionné sur le noyau intérieur 12. Lorsque l'on rapporte l'enveloppe extérieure 14 sur le noyau 12 muni de l'élément élastomère 16', les premiers moyens d'appui 27 déforment l'élément élastomère 16', de façon qu'il prenne la position illustrée sur la figure 2a, à savoir la position anti-retour. Dans cette position, du fait du rappel élastique de la paroi périphérique 42 de l'élément élastomère 16' contre les surfaces d'appui 36', du liquide ne peut pas s'infiltrer à l'intérieur de la zone étanche du dispositif 10.

On comprendra que le fonctionnement du dispositif 10 de la figure 2a est analogue à celui de la figure 1a.

On notera que, sur le dispositif des figures 2a, 2b, les moyens d'appui 27 sont davantage saillants que les moyens d'appui 27 des figures 1a à 1c, et que l'évidement 40 prolongeant l'orifice 34 a une hauteur relativement importante, plus importante que la hauteur de l'évidement 40 de la figure 1a. Il en résulte que l'élément élastomère 16' de la figure 2a est relativement éloigné de l'orifice 34, et qu'il ne risque donc pas de boucher cet orifice 34, même lorsque les dimensions des éléments 12 ou 14 varient du fait de tolérances de fabrication.

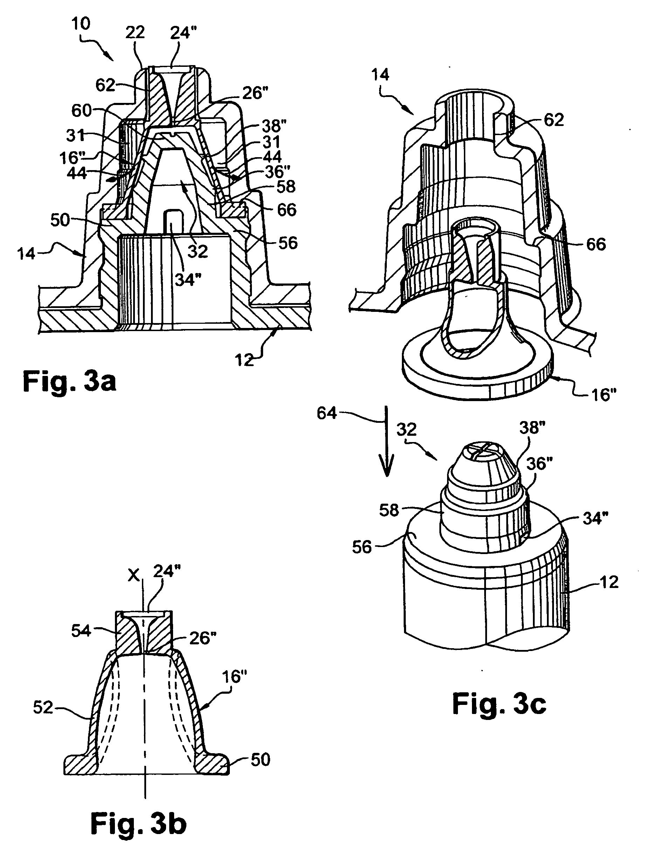

Sur le mode de réalisation des figures 3a à 3c conforme à l'invention, l'élément élastomère 16" a une forme générale de cône incurvé vers le haut, assimilable à la forme d'un tipi. Par ailleurs, l'extrémité 32 du noyau intérieur 12 a une forme générale assimilable à un cône incurvé vers le haut, plus précisément une forme générale ogivale, sur laquelle l'élément élastomère 16' est destiné à être chaussé.

Plus précisément, l'élément élastomère 16" comprend une collerette inférieure 50, une paroi latérale 52, ayant une forme générale d'hyperboloïde lorsque l'élément 16" est dans sa position non déformée, incurvée vers l'axe X de l'élément 16" (représentée en pointillés sur la figure 3b), et une extrémité 54, prolongeant la paroi 52, et dans laquelle sont formés des moyens 24" de dosage du liquide à distribuer. On notera que, pour pouvoir chausser l'élément élastomère 16" sur le noyau 12, cet élément doit être réalisé dans un matériau relativement souple, et la paroi 52 doit être relativement fine.

Par ailleurs, l'extrémité 32 du noyau 12, destinée à être recouverte par l'élément élastomère 16", est munie d'une base inférieure périphérique 56, servant de base à une paroi latérale 58,bombée vers l'extérieur, cette paroi 58 étant munie d'un sommet 60. La paroi latérale 58 porte deux saillies circulaires 36", 38", similaires aux nervures 36, 38 de la figure 1c, et servant de moyens d'appui sur la paroi latérale 52 de l'élément élastomère. En outre, la paroi latérale 58 est munie, au voisinage de la base 56, de deux cavités 34", diamétralement opposées, servant d'orifices de libération du liquide hors du noyau 12.

L'enveloppe 14 de la figure 3a est légèrement différente de celle de la figure 1a. En effet, cette enveloppe n'est pas munie des moyens de dosage 24, du fait que des moyens de dosage 24" sont prévus directement sur l'élastomère 16". Ainsi, l'extrémité 22 de l'enveloppe 14 définit une cavité tubulaire 62, laissant la place à l'extrémité 54 de l'élément élastomère.

L'assemblage du dispositif de la figure 3a se fait de façon analogue à l'assemblage des dispositifs des figures précédentes. On rapporte tout d'abord l'élément élastomère 16" sur le noyau 12, en le chaussant sur l'extrémité 32, dans la direction indiquée par la flèche 64. Le fait de rapporter l'élément 16" par-dessus le noyau intérieur 12 déforme sa paroi latérale 52. En effet, l'élément élastomère 16" dans sa position non déformée ayant un diamètre intérieur inférieur au diamètre extérieur de l'extrémité 32, est déformé par cette extrémité 32. Ainsi, un peu comme pour une chaussette, l'élément 16" se déforme et prend ainsi une position finale correspondant à la position anti-retour illustrée sur la figure 3a. En particulier, les moyens d'appui 36", 38" exercent une pression sur la paroi latérale 52 de l'élément élastomère 16", de façon à le déformer. Une fois l'élément 16" disposé sur le noyau 12, on peut rapporter l'enveloppe extérieure 14, en l'assemblant de la même façon que sur les figures 1a ou 2a sur le noyau 12.

On notera que, dans ce mode de réalisation, un épaulement 66 réalisé à l'intérieur de l'enveloppe extérieure 14 permet de pincer la collerette inférieure 50 de l'élément élastomère 16" contre la base 56 du noyau 12. Ce pincement permet de fixer l'élément élastomère 16" autour de l'extrémité 32, et d'assurer ainsi une étanchéité statique entre le noyau 12 et l'enveloppe 14. Par cette étanchéité statique, l'élément élastomère 16" empêche que du liquide, sortant des cavités 34" et s'écoulant entre la surface extérieure de l'extrémité 32 et la surface intérieure de l'élément élastomère 16", s'infiltre entre le noyau 12 et l'enveloppe 14. Pour assurer cette étanchéité statique, la partie 50 de l'élément élastomère 16" prend une unique position d'étanchéité tout au long de l'utilisation du dispositif 10. On notera que l'étanchéité statique se distingue de l'étanchéité dynamique réalisée par la paroi latérale 52 de l'élément élastomère 16", l'étanchéité dynamique autorisant la libération de liquide dans un sens.

Le fonctionnement du dispositif de la figure 3a va à présent être décrit. Ce fonctionnement est analogue à celui des figures précédentes. Lorsque le dispositif 10 est au repos, c'est-à-dire lorsque l'utilisateur n'appuie pas sur le réservoir, l'élément élastomère 16" est en position anti-retour, illustrée sur la figure 3a, de façon à empêcher que du liquide s'infiltre à l'intérieur de la zone étanche. Dans cette position, la paroi latérale 52, déformée notamment par les moyens d'appui 36", 38", est plaquée contre ces derniers, de façon à empêcher le retour de liquide. Lorsque l'utilisateur presse sur le réservoir, du liquide sort du noyau 12 par les orifices de libération 34". La pression exercée par le liquide déforme davantage la paroi latérale 52 de l'élément élastomère 16", dans le sens indiqué par les flèches 44 sur la figure 3a, si bien qu'un espace est créé entre les moyens d'appui 36", 38" et la paroi latérale 52, pour que du liquide puisse passer. Le liquide ainsi libéré peut ensuite s'écouler à l'intérieur de l'orifice 26" et des moyens de dosage 24". Lorsque suffisamment de liquide remplit les moyens de dosage 24", le liquide est distribué hors du dispositif 10. Une fois que l'utilisateur arrête d'exercer une pression sur le réservoir, le liquide n'exerce plus de pression sur la paroi latérale 52 de l'élément élastomère, si bien que celui-ci se plaque à nouveau contre les moyens d'appui 36", 38", et reprend donc sa position anti-retour.

On notera que l'invention n'est pas limitée aux modes de réalisation précédemment décrits. En particulier, les moyens d'appui peuvent prendre une multiplicité de forme, par exemple la forme de plots d'appui, tels que les plots 27, la forme de nervures d'appui circulaires, telles que les nervures 36, 38, 36", 38", ou encore la forme de surfaces d'appui, relativement étendues, telles que la surface d'appui 36'. Ces moyens d'appui peuvent être inter-changés ou combinés sur l'un quelconque des dispositifs décrits ci-dessus. Par ailleurs, on peut réaliser des nervures d'appui directement sur l'élément élastomère 16, 16' ou 16". Par exemple, on peut remplacer les nervures 36, 38 de la figure 1a par une surface plane, et prévoir des nervures circulaires, similaires aux nervures 36, 38, sur la surface inférieure 15 de l'élément élastomère 16, en les réalisant d'une seule pièce avec cet élément 16. On peut procéder de façon similaire sur les dispositifs des autres figures.

Pour ce qui concerne l'assemblage du dispositif 10, on peut prévoir que la première étape, dans laquelle l'élément élastomère 16, 16', 16" est rapporté sur le noyau 12, soit remplacée par une étape dans laquelle l'élément élastomère 16, 16', 16" est rapporté à l'intérieur de l'enveloppe 14, laquelle peut être disposée à l'envers pour maintenir en place l'élément élastomère..

On peut en outre envisager de diminuer le nombre de pièces du dispositif et les étrapes d'assemblage, en réalisant l'élément élastomère 16, 16', 16" par surmoulage ou par bi-injection, directement sur l'enveloppe 14 ou sur le noyau 12.

Par ailleurs, le dispositif 10 peut être rapporté directement sur un réservoir, ou bien sur un réservoir muni d'une pompe de distribution du liquide. On peut également prévoir que le réservoir soit réalisé d'une seule pièce avec le noyau 12.

Les avantages du dispositif 10 ont été présentés ci-dessus. On notera en particulier que la présence d'une zone évidée 31, 40 au droit des moyens d'appui laisse un dégagement autorisant la déformation de l'élément élastomère 16, 16', 16", afin qu'il prenne sa position anti-retour ou sa position de libération. Ainsi, la déformation de l'élément élastomère permet de le faire fléchir, et se distingue d'une seule compression contre une surface.

## Revendications

1. Dispositif (10) de distribution de liquide contenu dans un réservoir, comportant une enveloppe extérieure (14), un noyau intérieur (12) et un élément élastomère (16") intercalé entre le noyau intérieur (12) et l'enveloppe extérieure (14) et pouvant prendre :
- une position de libération de liquide, laissant sortir du liquide hors du dispositif (10), et
- une position anti-retour empêchant le retour de liquide à l'intérieur du dispositif,
l'élément élastomère (16") étant chaussé autour du noyau intérieur (12)
le dispositif comportant en outre des moyens (36", 38") d'appui sur l'élément élastomère (16"), au droit d'une zone évidée (31, 40), assurant la déformation de l'élément élastomère (16") pour qu'il prenne sa position anti-retour **caractérisé en ce que** les moyens d'appui comprennent une saillie (36", 38") ménagée sur le noyau intérieur (12) sur lequel est rapporté l'élément élastomère (16") et élément élastomère (16") est fixé autour de l'extrémité (32) du noyau intérieur (12), de façon à assurer une étanchéité statique entre le noyau intérieur (12) et l'enveloppe extérieure (14) du dispositif (10).

2. Dispositif selon la revendication précédente, dans lequel l'élément élastomère (16") dans sa position de libération de liquide subit une déformation, correspondant à une amplification de la déformation assurée dans sa position anti-retour.

3. Dispositif selon l'une quelconque des revendicatonss précédentes, dans lequel l'élément élastomère (16") comprend un cône incurvé vers le haut.

4. Dispositif selon l'une quelconque des revendications précédentes, comportant un canal de guidage du liquide, guidant le liquide libéré par l'élément élastomère hors du dispositif.

5. Dispositif selon l'une quelconque des revendications précédentes, dans lequel l'élément élastomère (16") comporte des moyens (24") de dosage du liquide à distribuer.

6. Dispositif selon l'une quelconque des revendications précédentes, dans lequel l'élément élastomère (16") est fixé autour de l'extrémité (32) par pincement.

7. Dispositif selon l'une Quelconque des revendications précédentes, dans lequel l'élément élastomère (16") comprend une collerette inférieure (50), pincée contre une base inférieure périphérique (56) de l'extrémité (32) du noyau intérieur (12).

8. Dispositif selon la revendication précédente, comprenant un épaulement (66) réalisé à l'intérieur de l'enveloppe extérieure (14), permettant de pincer la collerette inférieure contre la base (56) du noyau intérieur (12).

9. Dispositif selon l'une quelconque des revendications précédentes, dans lequel l'enveloppe extérieure (14) comprend des moyens (28) d'assemblage sur le noyau intérieur (12), par exemple par encliquetage serré, par serrage ou par soudure aux ultrasons.

## Claims

1. A device (10) for dispensing liquid contained in a container, said device including an outer envelope (14), an inner core (12) and an elastomer element (16") interposed between the inner core (12) and the outer enveloppe (14) and that can take up:
• a liquid release position allowing liquid to flow out of the device (10); and
• a non-return position preventing liquid from flowing back into the device;
the elastomer element (16") being fitted around the inner core (12), said device further including bearing means (36", 38") bearing against the elastomer element (16") in register with a recessed zone (31, 40), serving to deform the elastomer element (16") so that it can take up its non-return position **characterized in that** the bearing means comprise a projection (36", 38") formed on the inner core (12) on which the elastomer element (16") is fitted, and
the elastomer element (16") is secured around the end (32) of the inner core (12) to provide a static sealing between the inner core (12) and the outer envelope (14) of the device (10).

2. A device according to the preceding claim, wherein the elastomer element (16") in its liquid release position is subjected to a deformation corresponding to an amplification of the deformation to which it is subjected in its non-return position.

3. A device according to any preceding claim, wherein the elastomer element (16") comprises a cone that is curved upwards.

4. A device according to any preceding claim, including a liquid guide channel that guides the liquid released by the elastomer element out of the device.

5. A device according to any preceding claim, wherein the elastomer element (16") is provided with metering means (24") for metering out the liquid to be dispensed.

6. A device according to any preceding claim, wherein the elastomer element (16") is secured around the end (32) by pinching.

7. A device according to any preceding claim, wherein the elastomer element (16") comprises a lower collar (50) pinched against a peripheral lower base (56) of the end (32) of the inner core (12).

8. A device according to the preceding claim, comprising a shoulder (66) formed in the outer envelope (14) for pinching the lower collar (50) against the base (56) of the inner core (12).

9. A device according to any preceding claim, wherein the outer envelope (14) comprises means (28) for assembly on the inner core (12), for example, by tight snap-fastening, by clamping or else by ultrasonic welding.

## Patentansprüche

1. Vorrichtung (10) zur Ausgabe von in einem Tank enthaltener Flüssigkeit, mit einer äußeren Hülle (14), einem inneren Kern (12) und einem Elastomerelement (16"), das zwischen dem inneren Kern (12) und der äußeren Hülle (14) eingefügt ist und einnehmen kann:
- eine Flüssigkeitsfreigabeposition, die Flüssigkeit aus der Vorrichtung (10) hinauslässt, und
- eine Rückflusssperrposition, die den Rückfluss von Flüssigkeit ins Innere der Vorrichtung verhindert,
wobei das Elastomerelement (16") um den inneren Kern (12) gelegt ist, wobei die Vorrichtung weiterhin Mittel (36", 38") zur Anlage am Elastomerelement (16"), in genauer Entsprechung zu einem ausgehöhlten Bereich (31, 40), aufweist, die die Verformung des Elastomerelements (16") gewährleisten, damit es seine Rückflusssperrposition einnimmt, **dadurch gekennzeichnet, dass** die Mittel zur Anlage einen Vorsprung (36", 38") umfassen, der auf dem inneren Kern (12) angeordnet ist, auf den das Elastomerelement (16") gefügt ist, und dass das Elastomerelement (16") um das Ende (32) des inneren Kerns (12) befestigt ist, um eine statische Dichtung zwischen dem inneren Kern (12) und der äußeren Hülle (14) der Vorrichtung (10) zu gewährleisten.

2. Vorrichtung nach dem vorstehenden Anspruch, wobei das Elastomerelement (16") in seiner Flüssigkeitsfreigabeposition eine Verformung erfährt, die einer Verstärkung der in seiner Rückflusssperrposition gewährleisteten Verformung entspricht.

3. Vorrichtung nach einem der vorstehenden Ansprüche, wobei das Elastomerelement (16") einen nach oben gekrümmten Kegel umfasst.

4. Vorrichtung nach einem der vorstehenden Ansprüche, mit einem Kanal zur Leitung der Flüssigkeit, der die vom Elastomerelement freigegebene Flüssigkeit aus der Vorrichtung hinausleitet.

5. Vorrichtung nach einem der vorstehenden Ansprüche, wobei das Elastomerelement (16") Mittel (24") zur Dosierung der auszugebenden Flüssigkeit aufweist.

6. Vorrichtung nach einem der vorstehenden Ansprüche, wobei das Elastomerelement (16") um das Ende (32) durch Klemmen befestigt ist.

7. Vorrichtung nach einem der vorstehenden Ansprüche, wobei das Elastomerelement (16") einen unteren Kragen (50) umfasst, der gegen ein peripheres unteres Basisteil (56) des Endes (32) des inneren Kerns (12) geklemmt ist.

8. Vorrichtung nach dem vorstehenden Anspruch, die einen im Inneren der äußeren Hülle (14) ausgebildeten Absatz (66) umfasst, der es ermöglicht, den unteren Kragen (50) gegen das Basisteil (56) des inneren Kerns (12) zu klemmen.

9. Vorrichtung nach einem der vorstehenden Ansprüche, wobei die äußere Hülle (14) Mittel (28) zur Anbringung auf dem inneren Kern (12) umfasst, beispielsweise durch enge Rastverbindung, durch Festklemmen bzw. -spannen oder durch Ultraschallschweißen.
